# EUROPEAN PATENT APPLICATION

(11) **EP 1 386 929 A1**
(43) Date of publication of application: **04.02.2004**
(21) Application number: 02017058.5
(22) Date of filing: 29.07.2002
(51) Int. Cl.: C07K 14/47, A61K 38/17

(54) **Peptides associated with tumors and uses related thereto**

(71) Applicant: MTM Laboratories AG, 69120 Heidelberg (DE)
(72) Inventor: Ridder, Rüdiger, 69198 Schriesheim (DE); Martin, Peter, 69251 Gaiberg (DE); Rudy, Wolfgang, 75015 Bretten (DE)

(57) **Abstract**

The present invention relates to peptides derived from cell cycle regulatory proteins, the expression of which is altered in association with tumors in individuals. These peptides according to the present invention may be used for detection and therapy of tumors. For detection purposes the peptides may for example be used to detect antibodies directed against said peptides. In therapeutic respect the peptides may be used for immunotherapy or vaccination approaches. In therapeutic and diagnostic respect the peptides may be used in combination with one or more peptides derived from tumor associated proteins.

SEQUENCE LISTING

<110>  MTM Laboratories AG

<120>  Peptides associated with tumors and uses related thereto

<130>  arfp14vac/P/EP

<160>  23

<170>  PatentIn version 3.1

<210>  1
<211>  15
<212>  PRT
<213>  Artificial Sequence

<220>
<223>  Description of the artificial sequence: HLA restricted peptide

<400>  1

Gly His Asp Asp Gly Gln Arg Pro Ser Gly Gly Ala Ala Ala Ala
1               5                   10                  15


<210>  2
<211>  15
<212>  PRT
<213>  Artificial Sequence

<220>
<223>  Description of the artificial sequence: HLA restricted peptide

<400>  2

Val Val His Ile Pro Arg Leu Thr Gly Glu Trp Ala Ala Pro Gly
1               5                   10                  15


<210>  3
<211>  15
<212>  PRT
<213>  Artificial Sequence

<220>
<223>  Description of the artificial sequence: HLA restricted peptide

<400>  3

Val His Ile Pro Arg Leu Thr Gly Glu Trp Ala Ala Pro Gly Ala
1               5                   10                  15


<210>  4
<211>  15
<212>  PRT
<213>  Artificial Sequence

<220>
<223>  Description of the artificial sequence: HLA restricted peptide

<400>  4

Arg Arg Pro Gly His Asp Asp Gly Gln Arg Pro Ser Gly Gly Ala
1               5                   10                  15


<210>  5
<211>  9
<212>  PRT
<213>  Artificial Sequence

<220>
<223>  Description of the artificial sequence: HLA restricted peptide

<400>  5

His Asp Asp Gly Gln Arg Pro Ser Gly
1               5


<210>  6
<211>  9
<212>  PRT
<213>  Artificial Sequence

<220>
<223>  Description of the artificial sequence: HLA restricted peptide

<400>  6

Ala Ala Glu Leu Gly Pro Gly Gly Gly
1               5


<210>  7
<211>  9
<212>  PRT
<213>  Artificial Sequence

<220>
<223>  Description of the artificial sequence: HLA restricted peptide

<400>  7

Arg Arg Cys Pro Gly Gly Leu Pro Gly
1               5


<210>  8
<211>  9
<212>  PRT
<213>  Artificial Sequence

<220>
<223>  Description of the artificial sequence: HLA restricted peptide

<400>  8

Gly His Asp Asp Gly Gln Arg Pro Ser
1               5

<210>  9
<211>  9
<212>  PRT
<213>  Artificial Sequence

<220>
<223>  Description of the artificial sequence: HLA restricted peptide

<400>  9

Gly Gly Glu Asn Met Val Arg Arg Phe
1               5


<210>  10
<211>  9
<212>  PRT
<213>  Artificial Sequence

<220>
<223>  Description of the artificial sequence: HLA restricted peptide

<400>  10

Gln Leu Arg Arg Pro Arg His Ser His
1               5


<210>  11
<211>  9
<212>  PRT
<213>  Artificial Sequence

<220>
<223>  Description of the artificial sequence: HLA restricted peptide

<400>  11

Met Val Arg Arg Phe Leu Val Thr Leu
1               5


<210>  12
<211>  9
<212>  PRT
<213>  Artificial Sequence

<220>
<223>  Description of the artificial sequence: HLA restricted peptide

<400>  12

Val Leu Met Leu Leu Arg Ser Gln Arg
1               5


<210>  13
<211>  9
<212>  PRT

<213>  Artificial Sequence

<220>
<223>  Description of the artificial sequence: HLA restricted peptide

<400>  13

Arg Leu Gly Gln Gln Pro Leu Pro Arg
1               5


<210>  14
<211>  9
<212>  PRT
<213>  Artificial Sequence

<220>
<223>  Description of the artificial sequence: HLA restricted peptide

<400>  14

Arg Val Arg Val Phe Val Val His Ile
1               5


<210>  15
<211>  9
<212>  PRT
<213>  Artificial Sequence

<220>
<223>  Description of the artificial sequence: HLA restricted peptide

<400>  15

Thr Leu Arg Ile Arg Arg Ala Cys Gly
1               5


<210>  16
<211>  9
<212>  PRT
<213>  Artificial Sequence

<220>
<223>  Description of the artificial sequence: HLA restricted peptide

<400>  16

Arg Leu Thr Gly Glu Trp Ala Ala Pro
1               5


<210>  17
<211>  9
<212>  PRT
<213>  Artificial Sequence

<223>  Description of the artificial sequence: HLA restricted peptide

<400>  17

Met Val Arg Arg Phe Leu Val Thr Leu
1               5

<210>  18
<211>  9
<212>  PRT
<213>  Artificial Sequence

<220>
<223>  Description of the artificial sequence: HLA restricted peptide

<400>  18

Ala Ala Val Ala Leu Val Leu Met Leu
1               5

<210>  19
<211>  9
<212>  PRT
<213>  Artificial Sequence

<220>
<223>  Description of the artificial sequence: HLA restricted peptide

<400>  19

Ala Val Ala Leu Val Leu Met Leu Leu
1               5

<210>  20
<211>  9
<212>  PRT
<213>  Artificial Sequence

<220>
<223>  Description of the artificial sequence: HLA restricted peptide

<400>  20

His Ile Met Gly Arg Gly Arg Cys Val
1               5

<210>  21
<211>  9
<212>  PRT
<213>  Artificial Sequence

<220>
<223>  Description of the artificial sequence: HLA restricted peptide

<400>  21

Leu Met Leu Leu Arg Ser Gln Arg Leu
1               5

<210>  22
<211>  9
<212>  PRT
<213>  Artificial Sequence

<220>
<223>  Description of the artificial sequence: HLA restricted peptide

<400>  22

Ala Ala Pro Gly Ala Pro Ala Ala Val
1               5


<210>  23
<211>  9
<212>  PRT
<213>  Artificial Sequence

<220>
<223>  Description of the artificial sequence: HLA restricted peptide

<400>  23

Gly Ala Pro Ala Ala Val Ala Leu Val
1               5

## Description

The present invention relates to peptides derived from cell cycle regulatory proteins, the expression of which is altered in association with tumors in individuals. These peptides according to the present invention may be used for detection and therapy of tumors. For detection purposes the peptides may for example be used to detect antibodies directed against said peptides. In therapeutic respect the peptides may be used for immunotherapy or vaccination approaches. In therapeutic and diagnostic respect the peptides may be used in combination with one or more peptides derived from tumor associated proteins.

Each year several million people get affected by cancer and die from the disease. Despite intense research effort the mortality remained unchanged over the past years. In common practice patients suffering from carcinomas are treated by surgical removal of tumors and by chemo- or radio-therapeutic adjuvant therapy. These therapeutic means exhibit immense side effects, cause therapy related diseases, which in some patients even lead to death..

It would thus be desirable to provide a method for treatment of carcinomas, that could be applied in early stages of the disorders or may even be used for prevention of cancers. An especially preferred treatment for cancer should exactly target affected tissues and cells in order to specifically target the tumor cells and to reduce the side effects for unaffected tissues. Thereby the mortality of cancer patients could be probably reduced.

A promising approach for therapy of carcinomas could be based on removal of affected cells or tissues by the immune system of individuals. Analogous to the way the immune system is able to fight infections by micro-organisms etc. certain properties of transformed tumor cells and tissues may be used to elicit an immune response directed against these cells and tissues.

It is well known in the art that tumors are associated with alterations in molecular properties in cells. Commonly tumors are accompanied by the expression of altered polypeptides or by the expression of non-wild type levels of proteins. This is especially true for cell cycle regulatory proteins.

During the experiments leading to the present invention the inventors have found, that peptides derived from cell cycle regulatory proteins, which are expressed in association with tumors, may be used for detection and therapy of tumors. Especially useful are the expression products encoded by the INK4a gene locus.

The expression products encoded by the lNk4a locus are known to show non wild-type expression patterns in association with cancers. It is especially known, that the 16 kDa product p16^{INK4a} encoded by said gene is either overexpressed or not at all expressed in a large number cancers. Furthermore it has been reported, that other expression product encoded by that locus by an alternative reading frame such as p14ARF, which also functions as a cell cycle regulatory protein, albeit as member of a different regulatory pathway, is also overexpressed in association with cancers.

The inventors of the compounds and methods disclosed herein could show, that immunogenic peptides may be derived from the expression products encoded by the lNK4a locus. These products furthermore may be used to elicit a specific immune response directed against tumor cells expressing the lNK4a encoded gene products.

The subject matter specified in the claims of the present patent application is suitable to overcome the disadvantages of therapy and diagnosis of cancers as present in the art. The inventors identified a number of particular peptides, which are suitable for the generation of immune response against the tumor associated proteins and their degradation products. These immunogenic peptides known to show non-wild type expression in cells affected by tumors provide useful tools for the therapy and diagnosis of said diseases.

The present invention in one aspect relates to peptides and nucleic acids encoding these peptides, which derived from tumor associated proteins, and which may be used to elicit an immune response against said tumors. The peptides are derived from cell cycle regulatory proteins, which show non wild-type expression in association with tumors.

A second aspect of the present invention is a method for treatment of tumors using the peptides and nucleic acids disclosed herein. The method is based on the generation of an immune response in individuals directed against cells expressing the respective peptides. The method may be applied as preventive or therapeutic means to fight tumors.

A third aspect of the present invention is a method for diagnosis of tumors based on the detection of antibodies directed against the peptides disclosed herein in individuals. The detection of the antibodies may for example be performed using the disclosed peptides. Otherwise the antibodies may be detected using specific antibodies directed against them.

A fourth aspect of the present invention relates to a pharmaceutical composition for use in the therapeutic method according to the present invention. The pharmaceutical composition is characterized, in that it contains at least one of the peptides disclosed herein. Furthermore the pharmaceutical composition may copmprise one or more further peptides derived from a protein, that is associated with cancer.

A fifth aspect of the present invention relates to a kit for the detection of the antibodies directed against the inventive peptides in individuals. The kit may for example contain the respective peptides or antibodies for the detection of the antibodies. The kit may for example be used for diagnostic purposes to detect the presence or absence of a tumor or in the course of immunotherapy to monitor the therapeutic effect. Furthermore a kit according to the present invention may also comprise positive and/or negative control samples.

The present invention provides peptides and nucleic acids coding therefore, which are expressed in association with tumors, for the use in therapy and diagnosis of disorders such as cancer. The peptides are derived from cell cycle regulatory proteins, which show an altered expression in association with cancer.

The lNK4a gene locus as used in the context of the present invention is located on chromosome 9p21. The locus is alternatively nominated e.g. the lNK4a/ARF locus, CDKN2A gene, the MTS1 locus. The gene locus dealt with herein is that gene locus, that in one aspect encodes the gene product p16^{INK4a} protein, but is also known to encode for other gene product in alternative reading frames.

Altered expression as used in the context of the present invention may comprise for example any non wild-type expression of a nucleic acid. The expression may differ from the wild type expression either in the level of the expression (elevated expression level or reduced expression level) or the expression product (mutated expression product). A mutated expression product differs from the wild type expression product in sequence or in any structural parameter such as modification etc. The wild-type expression shall be understood to be the expression found in a normal control tissue.

An expression product according to the present invention may be any molecule transcribed from a gene or any molecule translated from such a transcript. Thus expression product as used in the context of the present invention may comprise polynucleotides such as e.g. DNA or RNA and polypeptides such as proteins, proteoglycans, peptides etc. Expression product as used in the context of the present invention shall comprise any transcript of a gene locus in forward or reverse direction including any reading frames, splicing variants or alternative promoters. Expression products as used herein shall thus comprise any alternative products encoded by the nucleic acids of a particular gene locus.

In one embodiment of the invention the cell cycle regulatory proteins are encoded by the lNk4a gene. The cell cycle regulatory proteins as disclosed herein comprise any proteins encoded by alternative reading frames, arising from alternative splicing or usage of alternative promoter sequences of said gene. In one embodiment of the present invention the proteins exhibit molecular weights of about 5 to 40 kDa or any value in between and preferably of about 10 to 20 kDa or any value in between and most preferably of about 14 to about 19 kDa or any value in between respectively. The cell cycle regulatory proteins for use in the context of the present invention are different from p16lNK4a cyclin dependent kinase inhibitor in at least one property selected from a group consisting of molecular weight, amino acid sequence, biological function and immunoreactivity.

In one embodiment of the present invention the immunogenic peptides that may be derived from a protein may be predicted as described by Rammensee et al, "MHC ligands and peptide motifs: 1st listing", Immunogenetics 41, 178-228, 1995 and Rammensee, et al., "MHC ligands and peptide motifs" Bioscience 1997, which methods shall be included herein by reference. The methods are exemplified below in the examples.

Peptides derived from the cell cycle regulatory proteins as used in the context of the present invention may comprise the respective full length proteins, fragments thereof, or peptides of any length. In certain embodiments of the present invention the peptides derived from cell cycle regulatory proteins showing altered expression in association with cancers may be e.g. at least one or more peptides selected from the peptides given in Table 1.

In one embodiment of the present invention the peptides derived from the INK4a expression products are 15-mer peptides. The 15-mer peptides are for example selected from a group consisting of the peptides :

G H D D G Q R P S G G A A A A
V V H I P R L T G E W A A P G
V H I P R L T G E W A A P G A
R R P G H D D G Q R P S G G A

In this embodiment the peptides are preferably presented by MHC class II molecules.

In another embodiment the peptides may be HLA-A1 restricted nonamer polypeptides selected from a group consisting of the following:

H D D G Q R P S G

A A E L G P G G G

R R C P G G L P G

G H D D G Q R P S

G G E N M V R R F

In yet another embodiment the peptides are HLA-A3 restricted nonamers selected from a group consisting of:

QLRRPRHSH
MVRRFLVTL
VLMLLRSQR
RLGQQPLPR
RVRVFVVHI
TLRIRRACG

R L T G E W A A P

In one further embodiment the peptides may be HLA A*0201 restricted nonamers selected from a group comprising the following peptides: 
M V R R F L V T L

A A V A L V L M L

A V A L V L M L L

L M L L R S Q R L

H I M G R G R CV

A A P G A P A A V

G A P A A V A L V
 (Poly)peptides as used according to the present invention may comprise amino acid chains of any length, including full length proteins, wherein the amino acid residues are linked by covalent peptide bonds.

Tumor associated peptides as disclosed in the context of the present invention shall comprise polypeptides of lengths of at least 4 amino acids. The peptides may for example comprise 4 to 50 amino acids or any number of amino acids in between. In another embodiment of the present invention the peptides may comprise polypeptides with more than 50 amino acids. These polypeptides for use in the context of the present invention may for example comprise 50, 100, 500, 750, 1000 amino acids or any number of amino acids in between and may comprise proteins, or fragments thereof, and/or fusion- or chimeric proteins comprising on or more additional heterologous sequences. The additional sequences may be derived from the native protein or may be heterologous, and such sequences may (but need not) be immunoreactive and/or antigenic. As detailed below, such polypeptides may be isolated from tumor tissue or prepared by synthetic or recombinant means.

As used herein, a polypeptide exhibiting biological properties of the tumor associated peptides is understood to be a polypeptide having at least substantially the same immunogenic properties, i.e. is still capable of binding an antibody directed against said poplypeptide, e.g. comprises an immunogenic portion of said polypeptide.

Peptides disclosed herein are immunogenic polypeptides. This requires, that the polypeptides may stimulate immune responses in host organisms either in the form the polypeptides adopt in their natural environment and/or especially in the form the polypeptides adopt after processing by the cellular antigen processing and presenting machinery.

Immunogenic portion as used above is a portion of a protein, that is recognized by a B-cell and/or T-cell surface antigen receptor. The immunogenic portions comprise at least 4 amino acid residues, at least 10 amino acid residues or at least 15 amino acid residues of the protein disclosed herein. In one embodiment of the present invention, particular domains of the protein, such as for example transmembrane domains or N-terminal leader sequences have been deleted.

The immunogenic portions according to the present invention react with antisera or specific antibodies in the same or nearly same intensity as the native full length proteins. The immunogenic portions are generally identified using the techniques well known in the art. Possible techniques are for example screening of the polypeptides for the ability to react with antigen-specific antibodies, antisera and/or T-cell lines or clones.

In certain embodiments of the present invention polypeptides may comprise fusion or chimeric polypeptides containing sequences disclosed herein. Fusion proteins comprise the polypeptide according to the present invention together with any second and further polypeptides, such as e.g. one or more polypeptides of the same sequence or of another sequence. Heterologous polypeptides may comprise e.g. enzymes, receptor molecules, antigens, antigenic or immunogenic epitopes or fragments, antibodies or fragments thereof, signalling polypeptides or signal transducing polypeptides, labelled polypeptides etc.. The immunogenic protein may for example be capable of eliciting a recall response. Examples of such proteins include tetanus, tuberculosis and hepatitis proteins (see, for example, Stoute et al. New Engl. J. Med., 336:86-91 (1997)).

In one embodiment of the invention the fusion peptides may be constructed for enhanced detection or purification of the polypeptides, or of complexes of the polypeptides with the respective immunological entities according to the present invention. For the purpose of purification tags, such as e.g. his-tags, myc-tags etc. may be added to the polypeptides. For the purpose of detection antigenic portions, enzymes, chromogenic sequences etc. may be fused to the polypeptides. The fusion proteins of the present invention may (but need not) include a linker peptide between the first and second polypeptides.

A peptide linker sequence may be employed to separate the first and the second polypeptides by a distance sufficient to ensure, that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes.

Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Pat. No. 4,935,233 and U.S. Pat. No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

The term tumor associated (poly)peptides according to the present invention may also comprise variants of the native proteins. These variants may differ from the native protein in one or more alterations such as substitutions, deletions, additions and/or insertions. The immunoreactivity of the variants according to the present invention is not substantially diminished compared to the native proteins. In a preferred embodiment of the invention the immunoreactivity and or acitivity is diminished less than 50% and in a more preferred embodiment the immunoreactivity and or activity is diminished less than 20 % compared to the native polypeptides.

The variants according to the present invention comprise e.g. conservative substitutions, so that the amino acids changed are substituted for amino acids with similar properties. The properties concerned may include polarity, charge, solubility, hydrophobicity, hydrophilicity and/or amphipathic nature of the amino acid residues.

The variants according to the invention may also comprise additional terminal leader sequences, linkers or sequences, which enable synthesis, purification or stability of the polypeptides in an easier or more comfortable way.

Variants of the polypeptides according to the present invention may be produced by means of conventional molecular biological processes (see, e.g., Sambrook et al., 1989, Molecular Cloning, A Laboratory Manual, 2^{nd} edition Cold Spring Harbor Laboratory Press, Cold Spring Harbor, NY)

The tumor associated polypeptides disclosed herein may be generated by synthetic means. In particular, synthetic polypeptides having fewer than about 100 amino acids, and generally fewer than about 50 amino acids, may be generated using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain (see, for example, Merrifield, J. Am. Chem. Soc. 85:2149-2146, 1963). Equipment for automated synthesis of polypeptides is commercially available from suppliers such as Perkin Elmer/Applied BioSystems Division (Foster City, Calif.), and may be operated according to the manufacturer's instructions.

According to the present invention polypeptides may be represented by nucleic acids coding for said polypeptides. These nucleic acids may for example be used for the in situ expression of the respective polypeptides. For the purpose of expression of nucleic acids, the particular nucleic acids may be joined with suitable other nucleic acid sequences, that enable for the cloning and expression of said nucleic acids encoding the tumor associated polypeptides.

Nucleic acid molecules according to the present invention may comprise polynucleotides or fragments thereof. Preferred polynucleotides may comprise at least 20 consecutive nucleotides, preferably at least 30 consecutive nucleotides and more preferably at least 45 consecutive nucleotides of the sequences. The nucleic acids according to the present invention may also be complementary or reverse complementary to any of said polynucleotides. Polynucleotides may for example include single-stranded (sense or antisense) or double-stranded molecules, and may be DNA (genomic, cDNA or synthetic) or RNA. RNA molecules comprise as well HnRNA (containing introns) as mRNA (not containing introns). According to the present invention the polynucleotides may also be linked to any other molecules, such as support materials or detection marker molecules, and may, but need not, contain additional coding or non-coding sequences.

A nucleic acid sequence encoding one or more peptides of the present invention is constructed using known recombinant DNA techniques to assemble separate nucleic acid sequences encoding the first and second polypeptides into an appropriate expression vector. The 3' end of a nucleic acid sequence encoding the first polypeptide is ligated, with or without a peptide linker, to the 5' end of a nucleic acid sequence encoding the second polypeptide ensuring the appropriate reading frames of the sequences to permit mRNA translation of the two nucleic acid sequences into a single fusion protein that retains the biological activity (antigenicity) of both the first and the second polypeptides.

The nucleotide sequences according to the present invention may be joined to a variety of other nucleic acid sequences using the known recombinant DNA techniques. The sequences may for example be cloned into any of a variety of cloning vectors, such as plasmids, phagemids, lambda phage derivatives and cosmids. Furthermore vectors such as expression vectors, replication vectors, probe generation vectors and sequencing vectors may be joined with the sequences disclosed herein. Sequences of special interest, that could be cloned to the nucleic acids according to the present invention are for example non coding sequences and regulatory sequences including promoters, enhancers and terminators.

In a preferred embodiment polynucleotides may be formulated such, that they are able to enter mammalian cells and to be expressed in said cells. Such formulations are especially useful for therapeutic purposes. The expression of nucleic acid sequences in target cells may be achieved by any method known to those skilled in the art. The nucleic acids may for example be joined to elements that are apt to enable their expression in a host cell. Such elements may comprise promoters or enhancers, such as CMV-, SV40-, RSV-, metallothionein I- or polyhedrin-promotors respectively CMV- or SV40-enhancers. Possible methods for the expression are for example incorporation of the polynucleotides into a viral vector including adenovirus, adeno-associated virus, retrovirus, vaccinia virus or pox virus. Viral vectors for the purpose of expression of nucleic acids in mammalian host cells may comprise pcDNA3, pMSX, pKCR, pEFBOS, cDM8, pCEV4 etc.. These techniques are known to those skilled in the art.

Other formulations for administration in therapeutic purposes include colloidal dispersion systems such as for example macromolecule complexes, microspheres, beads, micelles and liposomes.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., supra) bases may be exchanged and natural or synthetic sequences may be added. In order to link the DNA fragments with each other adapters or linkers may be added to the fragments. Furthermore, manipulations may be performed that provide suitable cleavage sites or that remove superfluous DNA or cleavage sites. If insertions, deletions or substitutions are possible, in vitro mutagenesis, primer repair, restriction or ligation may be performed. As analysis method usually sequence analysis, restriction analysis and other biochemical or molecular biological methods may be used.

A nucleic acid sequence encoding a fusion protein of the present invention is constructed using known recombinant DNA techniques to assemble separate nucleic acid sequences encoding the first and second polypeptides into an appropriate expression vector. The 3' end of a nucleic acid sequence encoding the first polypeptide is ligated, with or without a peptide linker, to the 5' end of a nucleic acid sequence encoding the second polypeptide ensuring the appropriate reading frames of the sequences to permit mRNA translation of the two nucleic acid sequences into a single fusion protein that retains the biological activity of both the first and the second polypeptides.

The tumor associated polypeptides of the present invention, and nucleic acids encoding such polypeptides, may be isolated from tumor tissue using any of a variety of methods well known in the art. Nucleic acid sequences corresponding to a gene (or a portion thereof) encoding one of the inventive tumor polypeptides may be isolated from a tumor cDNA library using a subtraction technique. Partial nucleic acid sequences thus obtained may be used to design oligonucleotide primers for the amplification of full-length nucleic acid sequences from a human genomic nucleic acid library or from a tumor cDNA library in a polymerase chain reaction (PCR), using techniques well known in the art (see, for example, Mullis et al., Cold Spring Harbor Symp. Quant. Biol. 51:263, 1987; Erlich ed., PCR Technology, Stockton Press, NY, 1989). For this approach, sequence-specific primers may be designed based on the nucleotide sequences provided herein and may be purchased or synthesized.

The ligated nucleic acid sequences encoding the inventive polypeptides are operably linked to suitable transcriptional or translational regulatory elements known to the person skilled in the art. The regulatory elements responsible for expression of nucleic acid may be located e.g. 5' to the nucleic acid sequence encoding the first polypeptides, within the coding sequences or 3' to the nucleic acid sequences encoding the first or any further polypeptide. Stop codons required to end translation and transcription termination signals are present 3' to the nucleic acid sequence encoding the second polypeptide.

The (poly)peptides as claimed according to the present invention are isolated. This means that the molecules are removed from their original environment. Naturally occurring proteins are isolated if they are separated from some or all of the materials, which coexist in the natural environment. Polynucleotides are isolated for example if they are cloned into vectors.

In certain preferred embodiments, described in more detail below, the polypeptides disclosed herein are prepared in an isolated, substantially pure form (i.e., the polypeptides are homogenous as determined by amino acid composition and primary sequence analysis). Preferably, the polypeptides are at least about 90% pure, more preferably at least about 95% pure and most preferably at least about 99% pure. The substantially pure polypeptides may for example be employed in pharmaceutical compositions.

In certain embodiments of the present invention, especially for the detection of specific antibodies directed against tumor associated polypeptides, these polypeptides themselves may be employed. Immunogenic portions as used herein is a portion of a protein, that is recognized by a B-cell and/or T-cell surface antigen receptor. The immunogenic portions comprise at least 5 amino acid residues, more preferably at least 10 amino acid residues and most preferably at least 15 amino acid residues of the tumor associated polypeptides according to the present invention.

Immunogenic portions useful for the detection of specific antibodies may be provided as oligopeptides or as part of larger proteins. This is dependent on the embodiment of the invention. Where antibodies are to be detected, the antigenic epitopes may either be primary structures of polypeptides or the epitopes may be built by complex arrangements of tertiary structures of polypeptides. Concerning cells directed against specific tumor associated peptides the relevant immunogenic portions are merely short fragments of peptides with a length of about 10 - 20 amino acids. Thus depending on the particular detection method the immunogenic portions have to be chosen.

Another aspect of the present invention relates to binding agents, antibodies and immunological entities specifically recognizing the peptides derived from proteins associated with tumors. In this respect the present invention provides binding agents such as antibodies and antigen-binding fragments, that specifically bind to the peptides derived from lNK4a encoded proteins showing altered expression patterns in tumors.

The term binding agent comprises a variety of substances such as oligopeptides, antibodies, peptidomimetic molecules comprising antigen binding oligopeptides, nucleic acids, carbohydrates, organic compounds, etc.. Antibody according to the present invention relates to antibodies which consist of polyclonal antibodies, essentially of pooled monoclonal antibodies with different epitopic specifities, as well as distinct monoclonal antibody preparations. Monoclonal antibodies are made using an antigen containing fragments of the polypeptides of the invention by methods well known to those skilled in the art (see, e.g., Kohler et al., Nature 256 (1975), 495). As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immunoglobulin expression library. Moreover, antibodies of the present invention include chimerical, single chain, and humanized antibodies.

For certain purposes, e.g. diagnostic methods, the antibody or binding agent of the present invention may be detectably labelled, for example, with a radioisotope, a bioluminescent compound, a chemiluminescent compound, a fluorescent compound, a metal chelate, or an enzyme. Furthermore any method suitable for the detection of the intermolecular interaction may be employed.

The antibody or antigen-binding agent is said to react specifically, if it reacts at a detectable level with a peptide disclosed herein, and does not significantly react with other peptides. The antibodies according to the present invention may be monoclonal or polyclonal antibodies. Other molecules capable of binding specifically may be for example antigen-binding fragments of antibodies such as Fab fragments, RNA molecules or polypeptides. According to the present invention binding agents may be used isolated or in combination. By means of combination it is possible to achieve a higher degree of sensitivity.

The antibodies useful for the methods according to the present invention may comprise further binding sites for either therapeutic agents or other polypeptides or may be coupled to said therapeutic agents or polypeptides. Therapeutic agents may comprise drugs, toxins, radio-nuclides and derivatives thereof. The agents may be coupled to the binding agents either directly or indirectly for example by a linker or carrier group. The linker group may for example function in order to enable the coupling reaction between binding agent and therapeutic or other agent or the linker may act as a spacer between the distinct parts of the fusion molecule. The linker may also be cleavable under certain circumstances, so as to release the bound agent under said conditions. The therapeutic agents may be covalently coupled to carrier groups directly or via a linker group. The agent may also be non-covalently coupled to the carrier. Carriers that can be used according to the present invention are for example albumins, polypeptides, polysaccharides or liposomes.

The antibody used according to the present invention may be coupled to one or more agents. The multiple agents coupled to one antibody may be all of the same species or may be several different agents bound to one antibody.

The invention also relates to a transgenic non-human animal such as transgenic mouse, rats, hamsters, dogs, monkeys, rabbits, pigs, C. elegans and fish such as torpedo fish comprising a nucleic acid molecule or vector of the invention, preferably wherein said nucleic acid molecule or vector may be stably integrated into the genome of said non-human animal, preferably such that the presence of said nucleic acid molecule or vector leads to the expression of the inventive cell cycle regulatory protein derived polypeptide (or related polypeptide) of the invention, or may otherwise be transiently expressed within the non-human animal. Said animal may have one or several copies of the same or different nucleic acid molecules encoding one or several forms of the inventive polypeptide. This animal has numerous utilities, including as a research model for the development of therapies, treatment, etc. for tumors. Accordingly, in this instance, the non-human mammal is preferably a laboratory animal such as a mouse or rat.

It is one aspect of the present invention to detect tumors on the basis of the detection of immunological entities directed against the peptides disclosed herein.

The detection as used herein may also comprise the detection of immunological entities disclosed herein together with immunological entities directed against other tumor associated peptides. These peptides may e.g. comprise p13,5, p14, p15, p16, p19, p21, p27, p53, Rb protein, osteopontine, grp, renal dipeptidase, id1, mcm5, mcm2, cdc6, MDM2 .The peptides furthermore may for example be derived from peptides encoded by the genes disclosed in the documents WO9904265A2, WO0149716A2, WO0055633A2 and WO0142792A2, which shall be included by reference herein. In one embodiment of the invention a set of polypeptides may be used for the detection of antibodies. The set may be a combination of relevant peptides in solution, in cases, where information about the presence of immunological entities in general is sought. In contrast, when information about the presence or absence of particular peptides within a set of peptides is sought, the peptides may for example be tested each as a single, simultaneously in multiple testing reactions. Such experiments may for example be carried out in form of multi-well tests or using peptide arrays etc.

The method for detection of disorders according to the present invention thus may employ the detection of immunological entities directed against one single peptide or the detection of a set of immunological entities. The use of a multiplicity of potential peptides raises the probability to detect the presence of a particular disorder, and may furthermore give additional information useful in stratification of a disorder, in monitoring the disease course or in assessment of prognosis concerning the disease course.

Immunological entities as used in the context of the present invention shall comprise any components of the mammalian immune system, that are able to specifically react with an antigenic epitope. Such immunlogical entities may comprise for example antibodies, all immunoglobulins, such as e.g. lgG, lgM, IgA, lgE, lgD, specific CD8+ T-cells or specific T-helper cells.

Diagnosis as used in the context of the present invention may comprise determining the presence or absence and/or the level of specific immunological entities directed against particular tumor associated peptides in a sample, and assessing diagnosis from said presence or absence and/or level of immunological entities specifically directed against said tumor associated polypeptides.

Based upon the determined presence or absence and/or the levels of immunological entities specifically directed against particular tumor associated peptides in the samples individuals can be subdivided into subgroups. The subgroups may be created according to scientific or clinical data, such as e.g. survival, recurrence of disease, frequency of metastases etc., related to the presence or absence and/or levels of particular tumor associated peptides in samples of tissues affected with a particular disorder, of tissues being in question of being affected with a particular disorder or of tissues at risk of being affected with a particular disorder.

Based upon these subgroups an assessment of prognosis may be done. According to the subgroups the therapy of the individuals affected by the disorders (e.g. tumors) may be tailored.

Monitoring may comprise detecting the presence or absence and/or the level of immunological entities specifically directed against tumor associated (poly)peptides in samples taken at different points in time and determining the changes in said levels or presences or absences. According to said changes the course of the disease can be followed. E.g. the occurrence of immunological entities directed against tumor associated (poly)peptides, that have not been present at an earlier time-point may be indicative of the progression of evolution of the affected tissue. The course of the disease may be used to select therapy strategies for the particular individual.

Monitoring may also pertain to the monitoring of the therapeutic effect in the course of an immunotherapy according to the present invention'.

Another aspect of diagnosis and monitoring of the disease course according to the present invention may comprise the detection of minimal residual disease. This may comprise for example the detection of presence and/or level of immunological entities specifically directed against said tumor associated (poly)peptides, that have not been present in earlier examinations in one or more body samples following initial therapy of an individual once or at several timepoints. According to the presence and/or level of immunological entities specifically directed against tumor associated polypeptides detected in the samples one may select a suitable therapy for the particular individual.

Furthermore the diagnostic method may be carried out to detect disseminated tumor cells in biological samples as MRD diagnosis of minimal residual disease. For this purpose the detection of the level of immunological entities or the presence of immunological entities, specific for particular peptides, that have not been detected in prior examinations, may be performed.

A sample according to the method of the present invention may comprise any sample comprising immunological entities as defined above. Samples may comprise samples of clinical relevance, such as e.g. blood, serum, plasma, secretions, smears, body fluids, urine, semen, stool, sputum, bile, biopsies, cell-and tissue-samples. Biopsies as used in the context of the present invention may comprise e.g. resection samples of tumors, tissue samples prepared by endoscopic means or needle biopsies of organs.

Such samples may comprise for example intact cells, lysed cells or any liquids containing antibodies, immunoglobulins or cells specifically directed against tumor associated peptides. Even solids, to which cells, cell fragments or antigen binding polypeptides, such as antibodies or immunoglobulins may adhere, or may be fixed, may be samples according to the method disclosed herein. The method for detection of the level of the immunological entities according to the present invention is any method, which is suited to detect very small amounts of specific immunological entities in biological samples. The detection reaction according to the present invention is a detection either on the level of antibodies or on the level of cells specific for particular antigens.

The compositions and methods according to the present invention may be applied to any eukaryotic organism exhibiting an immunologic defense system. The eukaryotic organisms are for example animals of agricultrual value such as pigs, cows, sheep, etc., companion animals, such as cats, dogs, horses etc., animals employed in research purposes such as mice, rats, rabbits, hamsters etc. or humans.

The methods disclosed herein may be applied generally to proliferative disorders. Proliferative disorders according to the present invention comprise diseases characterized by abnormal growth properties of cells or tissues compared to the growth properties of normal control cells or tissues. The growth of the cells or tissues may be for example abnormally accelerated, decelerated or may be regulated abnormally. Abnormal regulation as used above may comprise any form of presence or absence of non wild-type responses of the cells or tissues to naturally occuring growth regulating influences. The abnormalities in growth of the cells or tissues may be for example neoplastic or hyperplastic.

In one embodiment the cell proliferative disorders are tumors. Tumors may comprise tumors of the head and the neck, tumors of the respiratory tract, tumors of the gastrointestinal tract, tumors of the urinary system, tumors of the reproductive system, tumors of the endocrine system, tumors of the central and peripheral nervous system, tumors of the skin and its appendages, tumors of the soft tissues and bones, tumors of the lymphopoietic and hematopoietic system etc.. Tumors may comprise for example neoplasms such as benign and malignant tumors, carcinomas, sarcomas, leukemias, lymhomas or dysplasias. In a preferred embodiment the tumor is for example cancer of the of the head and the neck, cancer of the respiratory tract, cancer of the gastrointestinal tract, cancer of the skin and its appendages, cancer of the central and peripheral nervous system, cancer of the urinary system, cancer of the reproductive system, cancer of the endocrine system, cancer of the soft tissues and bone, cancer of the hematopoietic and lymphopoietic system.

In certain embodiments of the invention the carcinoma is cervical cancer, colon cancer, gastric cancer, breast cancer, bladder cancer, lung cancer etc.

The detection may be carried out in solution or using reagents fixed to a solid phase. Solid phases may comprise beads of a variety of materials, such as e.g. agarose, dextrane polymers, polystyrene, silica, etc. or surfaces of suitable materials such as e.g. polystyrene, glass, agarose, protein, dextran etc. coated surfaces etc.. The detection of one or more immunological entities, such as immunoglobulins or cells carrying specific antigen recognizing epitopes, with different antigen binding speificities may be performed in a single reaction mixture or in two or more separate reaction mixtures. Alternatively the detection reactions for several immunological entities may for example be performed simultaneously in multi-well reaction vessels.

The immunological entities specifically recognizing particular tumor associated peptides may be detected using reagents that specifically recognise these immunological entities alone or in complex with their respective antigen (e.g. antibodies), or reagents, that are specifically recognized by the immunological entities themselves (e.g. the antigen). In one embodiment the antigen may be fused to another polypeptide, so as to allow binding of the antigen by the immunological entity in question and simultaneously binding of the second part of the fusion protein by another (labelled) antibody for the detection. The detection reaction for the immunological entities may comprise one or more reactions with detecting agents either recognizing the initial entities or recognizing the prior molecules used to recognize the immunological entities.

The detection reaction further may comprise a reporter reaction indicating the presence or absence and/or the level of the immunological entities. The reporter reaction may be for example a reaction producing a coloured compound, a bioluminescence reaction, a chemiluminescent reaction, a fluorescence reaction, generally a radiation emitting reaction etc.. The detection reactions may for example employ antibodies or binding reagents that are detectably labelled.

Furthermore the binding of detection molecules to the entities in question may be detected by any measurable changes in physical or physical-chemical properties, such as changes in spectroscopic properties, in magnetic resonance properties etc.. Different immunological entities or immunological entities of different specificities may be recognized by different methods or agents. This may be due to difficulties in detection of several entities, or entities with particular specificities by a particular method. An advantage of the use of different detection techniques for different immunological entities or for immunological entities with different specificities may for example be, that the different reporter signals referring to different immunological entities could be distinguished.

Generally in a method according to the present invention the detection of different immulogical entities such as the detection of immunoglobulins and the detection of immunocompetent cells may be performed simultaneously. The detection may be e.g. performed simultaneously in one vessel or in different reaction containers or samples. Furthermore the reaction according to the present invention may be performed sequentially one subsequent to the other.

For all detection purposes optionally the original sample may be concentrated by any suitable means known to those of ordinary skill in the art. Furthermore steps may be involved to selectively extract immunological entities from the sample mixture such as affinity based purification techniques either employing specific antibodies or the respective antigen recognized by the entities in question.

Applicable formats for the detection reaction according to the present invention may be, (reverse) blotting techniques, such as Western-Blot. The blotting techniques are known to those of ordinary skill in the art and may be performed for example as electro-blots, semidry-blots, vacuum-blots or dot-blots. Furthermore immunological methods for detection of molecules may be applied, such as for example immunoprecipitation or immunological assays, such as ELISA, RIA, Elispot assay, lateral flow assays, immuno-cytochemical methods etc..

In one preferred embodiment of the invention the detection of the level of immunological entities specific for tumor associated peptides is carried out on the level of antibodies. This may be e.g. performed using the specific interaction between the respective tumor associated peptides with the antibodies. The determination of the presence or absence and/or the level of the antibodies may for example be carried out with recombinantly produced tumor associated peptides. The peptides can be used in many different detection techniques for example in western-blot, ELISA or immuno-precipitation. In one embodiment the detection of antibodies is carried out as antibody capture assay (Antibodies A laboratory Manual, Harlow, Ed. et al., Cold Spring Harbor Laboratory 1988).

In another embodiment of the invention the detection of the specific antibodies is carried out using monoclonal or polyclonal antibodies specifically recognizing the antigen binding epitope of the first antibodies. For this purpose the above mentioned immunological detection procedures may be applied. In a further embodiment chimeric antigens may be employed in the detection reaction. Such chimeric antigens may for example comprise fusion proteins combining the antigenic epitope of a tumor associated polypeptide, recognized by the antibody in question, fused to another antigen, that may be recognized by a detection antibody. The particular antigens within the chimeric polypeptide may be separated by a linker or spacer region.

Any other method for determining the amount of particular antibodies or immunoglobulins in biological samples can be used according to the present invention.

Generally the detection of the antibodies according to the present invention may be performed as well in vitro as directly in situ for example in the course of an immuno-histochemical or immuno-cytochemical staining reaction.

Cells exhibiting specificity for a particular antigen may be detected by any methods suitable for that purpose known to those of ordinary skill in the art. Methods may for example comprise proliferation-assays, cytokine-ELISAs, ELlSpot assays, intracellular FACS-staining, PCR-mediated identification of peptide-specific cytokine (or similar) -expressing cells, tetramer-staining, cytotoxicity assays and DTH-(delayed type hypersensitivity) reactions.

In case of proliferation-assays induction of peptide-specific T-cell proliferation may be measured by methods known to those of skill in the art. This can be achieved by simple counting of cells, by measuring incorporation of labelled nucleotides into cellular DNA or by measuring level and/or activity of cellular protein(s). Cytokine-ELISA may comprise identification of peptide-specific cytokine-secreting cells by measuring cytokine levels in supernatant. In the course of an ELISpot assay the number of peptide-specific cytokine (i.e. IFN-g)- secreting cells in a sample is determined. Similarly the intracellular FACS-staining identifies cytokine-expressing cells on the protein level. In contrast (real-time) PCR may be used for identification of peptide-specific cytokine (or similar) -expressing cells on the transcript level. In the course of a tetramer-staining assay the label is a tetramer-molecule of recombinant MHC-class I molecules, loaded with specific peptide and coupled to a dye. The tetramer binds to the T-cell receptor. Cytotoxicity assays are a method for identification of cells, that can recognize and kill target cells in a peptide-specific manner. DTH-(delayed type hypersensitivity) reaction is based on the measuring of skin-reaction of vaccinated persons after intradermal (or similar) application of peptide(s).

The method for the detection of immunological entities as disclosed herein may be performed for the purpose of monitoring in the course of immunotherapeutic treatments of individuals. In this respect the presence or absence and or the level of antibodies directed against an inventive peptide in an individual is determined. The determination of the level may be performed using the methods as se forth above. The detection may be performed at several consecutive points in time as to monitor the timely alteration of the level of the immunological entities. The determination may for example be performed daily, weekly, monthly, once a year, or in once in a decade or at any interval in between.

Another aspect of the present invention is a testing kit for performing the method according to the present invention. The kit may be for example a diagnostic kit or a research kit.

A kit according to the present invention comprises at least an agent suitable for detecting the immunological entities according to the method disclosed herein. Furthermore a kit according to present invention may comprise:
a) reagents for the detection of the antibodies or cells specifically recognizing antigens.
b) reagents and buffers commonly used for carrying out the detection reactions as described herein, such as buffers, detection-markers, carrier substances and others
c) a sample for carrying out a positive control reaction, that may comprise an antigen or a set of antigens, to which all members of a target population of individuals have antibodies.

The reagent for the detection of the antibodies or cells specifically recognizing antigens may include any agent capable of binding to the antibodies or cells specifically recognizing antigens. Such reagents may include proteins, polypeptides, nucleic acids, peptide nucleic acids, glycoproteins, proteoglycans, polysaccharids or lipids.

The sample for carrying out a positive control may comprise for example an antigenic peptide or a set of antigenic peptides. Suitable antigens may include antigens, against which a wide percentage of the population has antibodies. Examples of such antigens may for example comprise antigens present in lysed E. coli cells, tetanus antigen, the viral capsid antigen of the Epstein-Barr virus, antigens derived from matrix proteins of Haemophilus influenzae. The antigens may for example be used as a mixture to ensure, that a particular individual actually displays a positive reaction.

In a preferred embodiment of the invention the detection of the immunological entities directed against particular tumor associated polypeptides is carried out on the level of antibodies. In this embodiment the binding agent may be for example a tumor associated polypeptide or a fragment thereof, recognized by the respective antibody, or a fusion polypeptide comprising said polypeptide or a fragment thereof. Furthermore the binding agent may comprise an antibody or a fragment thereof specific for the antibody in question, for the complex of the antibody with the respective tumor associated polypeptide or for an antigenic epitope fused to the polypeptide.

In an other embodiment of the test kit the detection of the immunological entities is carried out on the level of cells specifically recognizing tumor associated polypeptides. In this embodiment of the invention the reagent for the detection may be for example a tumor associated polypeptide or a fragment thereof, recognized by the respective antibody or T-cell receptor, or a fusion polypeptide comprising said tumor associated polypeptide or a fragment thereof. Furthermore the binding agent may comprise an antibody or a fragment thereof specific for the antibody in question, for the complex of the antibody with the respective polypeptide or for an antigenic epitope fused to the polypeptide.

The tumor associated peptides disclosed herein may be used in vaccines, that may comprise one or more tumor associated polypeptides as disclosed herein and may comprise optionally one or more further tumor associated peptides.

According to the present invention tumor associated polypeptides that comprise an immunogenic portion may be used for immuno-therapy for the treatment of cancer. Immunotherapy may be broadly classified into either active or passive immunotherapy. In active immunotherapy, treatment relies on the in vivo stimulation of the endogenous host immune system to react against tumours with the administration of immune response-modifying agents (for example, tumour vaccines, bacterial adjuvants, and/or cytokines). A patient may be afflicted with disease, or may be free of detectable disease. Accordingly, the compounds disclosed herein may be used to treat cancer or to inhibit the development of cancer. The compounds are preferably administered either prior to or following primary treatment of tumours such as surgical removal of the tumours, treatment by administration of radiotherapy and/or conventional chemotherapeutic drugs or any other mode of treatment of the respective cancer or its precursors.

In passive immunotherapy, treatment involves the delivery of biologic reagents with established tumour-immune reactivity (such as effector cells or antibodies) that can directly or indirectly mediate antitumour effects and does not necessarily depend on an intact host immune system. Examples of effector cells include T lymphocytes (for example, CD8+ cytotoxic T-lymphocytes, CD4+ T-helper, tumour-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Pat. No. 4,918,164), for passive immunotherapy.

The predominant method of procuring adequate numbers of T-cells for adoptive transfer immunotherapy is to grow immune T-cells in vitro. Culture conditions for expanding single antigen-specific T-cells to several billion in number with retention of antigen recognition in vivo are well known in the art. These in vitro culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic-, macrophage- or B-cells, may be pulsed with immunoreactive polypeptides or transfected with a nucleic acid sequence(s), using standard techniques well known in the art. For example, antigen presenting cells may be transfected with a nucleic acid sequence, wherein said sequence contains a promoter region appropriate for increasing expression, and can be expressed as part of a recombinant virus or other expression system. For cultured T-cells to be effective in therapy, the cultured T-cells must be able to grow and distribute widely and to survive long term in vivo. Studies have demonstrated that cultured T-cells can be induced to grow in vivo and to survive long term in substantial numbers by repeated stimulation with antigen supplemented with IL-2 (see, for example, Cheever, M., et al, "Therapy With Cultured T Cells: Principles Revisited," Immunological Reviews, 157:177, 1997).

According to the present invention sets of one or more tumor associated polypeptides may be employed to generate and/or isolate tumour-reactive T-cells, which can then be administered to the patient. In one technique, antigen-specific T-cell lines may be generated by in vivo immunization with short peptides corresponding to immunogenic portions of the disclosed polypeptides. The resulting antigen specific CD8+ CTL or CD4+ T-helper cells clones may be isolated from the patient, expanded using standard tissue culture techniques, and returned to the patient.

Alternatively, peptides corresponding to immunogenic portions of the polypeptides of the invention may be employed to generate tumour reactive T-cell subsets by selective in vitro stimulation and expansion of autologous T-cells to provide antigen-specific T-cells which may be subsequently transferred to the patient as described, for example, by Chang et al. (Crit. Rev. Oncol. Hematol., 22(3), 213, 1996). Cells of the immune system, such as T-cells, may be isolated from the peripheral blood of a patient, using a commercially available cell separation system, such as CellPro Incorporated's (Bothell, Wash.) CEPRATE.TM. system (see U.S. Pat. No. 5,240,856; U.S. Pat. No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T-cells. The population of tumour antigen-specific T-cells is then expanded using standard techniques and the cells are administered back to the patient.

In another embodiment, T-cell receptors and/or antibodies specific for the polypeptides can be cloned, expanded, and transferred into other vectors or effector cells for use in adoptive immunotherapy.

In a further embodiment, syngeneic or autologous dendritic cells may be pulsed with peptides corresponding to at least an immunogenic portion of a polypeptide disclosed herein. The resulting antigen-expressing and/or presenting dendritic cells may either be transferred into a patient, or employed to stimulate T-cells to provide antigen-specific T-cells which may, in turn, be administered to a patient. The use of peptide-pulsed dendritic cells to generate antigen-specific T-cells and the subsequent use of such antigen-specific T-cells to eradicate tumours in a murine model has been demonstrated by Cheever et al, (Immunological Reviews, 157:177, 1997).

Monoclonal antibodies directed against tumor associated peptides presented on cellular membranes may according to the present invention also be used as therapeutic compounds in order to diminish or eliminate tumours. The antibodies may be used on their own (for instance, to inhibit metastases) or coupled to one or more therapeutic agents. Suitable agents in this regard include radionuclides, differentiation inducers, drugs, toxins, and derivatives thereof. Preferred radionuclides include 90Y, 1231, 1251, 1311, 186Re, 188Re, 211 At, and 212Bi.

Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

Pharmaceutical compositions useful in immuno-therapy according to the present invention may comprise a set of one or more tumor associated polypeptides (or variants thereof), and a physiologically acceptable carrier. The pharmaceutical composition may for example comprise one or more of the inventive peptides derived from tumor associated proteins. In one embodiment of the present invention the pharmaceutical composition may additionally comprise one or more further peptides derived from other proteins, which show non-wild type expression in tumors. Such proteins may for example be cell cycle regulatory proteins, proteases, such as metallo-proteinases, signal transduction molecules, receptors, etc. In one embodiment the additional peptides are derived from cell cycle regulatory proteins such as p13,5, p14, p15, p16, p19, p21, p27, p53, Rb protein, osteopontine, grp, renal dipeptidase, id1, mcm5, mcm2, cdc6, MDM2 etc. The peptides disclosed herein may be used in combination of one or more further peptides associated with cancer.

Examples for peptides, that are associated with cancer are disclosed in WO9904265A2, WO0149716A2, WO0055633A2 and WO0142792A2, which documents shall be incorporated herein by reference. One or more of the peptides disclosed in this documents may be used for therapeutic and or diagnostic purposes in combination with one or more peptides disclosed herein.

The vaccines may additionally comprise a non-specific immune-response enhancer, wherein the non-specific immune response enhancer is capable of eliciting or enhancing an immune response to an exogenous antigen. Examples of non-specific-immune response enhancers include adjuvants, biodegradable microspheres (e.g., polylactic galactide) and liposomes (into which the polypeptide is incorporated). Pharmaceutical compositions and vaccines may also contain other epitopes of tumour antigens, either incorporated into a fusion protein or present within a separate polypeptide.

In one embodiment of the invention the pharmaceutical composition for use in immunotherapy may comprise further immunogenic molecules, which are known to effectively elicit immune responses. By this way the immune response to the target molecules may be enhanced as described in: Benoit, J.; et al., "T cell defined tumor antigens", Current Opinion in Immunology 9, 1997, 684-693 and: Markwin P., et. al., "Identification of Peptides for Immunotherapy of Cancer. It is still worth the effort", Critical Reviews in Immunology. 18, 1998, 7-27. Both cited articles shall be included here within by reference.

Alternatively, a pharmaceutical composition or vaccine suitable for immunotherapy according to the present invention may contain nucleic acids, that code for one or more tumor associated polypeptides according to the present invention. Nucleic acids may for example include single-stranded (sense or antisense) or double-stranded molecules, and may be DNA (genomic, cDNA or synthetic) or RNA. RNA molecules comprise as well HnRNA (containing introns) as mRNA (not containing introns). According to the present invention the polynucleotides may also be linked to any other molecules, such as support materials or detection marker molecules, and may, but need not, contain additional coding or non-coding sequences. The nucleic acid may be administered in a way that allows the polypeptides to be generated in situ. Suitable expression systems are known to those skilled in the art. The expression of the polypeptides may for example be persistent or transient. In pharmaceutical compositions and/or vaccines, providing for in-situ expression of polypeptides, the nucleic acids may be present within any suitable delivery system known to those of ordinary skill in the art, including nucleic acid expression systems, bacteria and viral expression systems. Appropriate nucleic acid expression systems comprise the necessary regulatory nucleic acid sequences for expression in the patient, such as suitable promoters, terminators etc..

Bacterial delivery systems may for example employ the administration of a bacterium that expresses an epitope of a cell antigen on its cell surface. In a preferred embodiment, the nucleic acid may be introduced using a viral expression system such as e.g., vaccinia virus, retrovirus, or adenovirus, which may involve the use of a non-pathogenic, replication competent virus. Suitable systems are known to those of ordinary skill in the art and are disclosed, for example, in Fisher-Hoch et al., PNAS 86:317-321, 1989; Flexner et al., Ann. N.Y. Acad Sci. 569:86-103, 1989; Flexner et al., Vaccine 8:17-21, 1990; U.S. Pat. Nos. 4,603,112, 4,769,330, and 5,017,487; WO 89/01973; U.S. Pat. No. 4,777,127; GB 2,200,651;

EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207, 1993.

In another embodiment transgenic mammalian cells may be used for delivery and/or expression of the nucleic acids. The methods for producing nucleic acid constructs suitable for in-situ expression of polypeptides are known to those of skill in the art.

Furthermore the nucleic acid may be administered as naked nucleic acids. In this case appropriate physical delivery systems, which enhance the uptake of nucleic acid may be employed, such as coating the nucleic acid onto biodegradable beads, which are efficiently transported into the cells. Administration of naked nucleic acids may for example be useful for the purpose of transient expression within a host or host cell.

The pharmaceutical compositions used for immuno-therapy according to the present invention may be administered by any suitable way known to those of skill in the art. The administration may for example comprise injection, such as e.g., intracutaneous, intramuscular, intravenous or subcutaneous injection, intranasal administration for example by aspiration or oral administration. A suitable dosage to ensure the pharmaceutical benefit of the treatment should be chosen according to the parameters, such as age, sex, body weight etc. of the patient, known to those of skill in the art.

The type of carrier to be employed in the pharmaceutical compositions of this invention, will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (e.g., polylactic glycolide) may also be employed as carriers for the pharmaceutical compositions of this invention. Suitable biodegradable microspheres are disclosed, for example, in U.S. Pat. Nos. 4,897,268 and 5,075,109. Any of a variety of immune-response enhancers may be employed in the vaccines of this invention.

For example, an adjuvant may be included. Most adjuvants contain a substance designed to protect the antigen from rapid catabolism, such as aluminum hydroxide or mineral oil, and a nonspecific stimulator of immune response, such as lipid A, Bordetella pertussis or Mycobacterium tuberculosis. Such adjuvants are commercially available as, for example, Freund's Incomplete Adjuvant and Complete Adjuvant (Difco Laboratories, Detroit, Mich.) and Merck Adjuvant 65 (Merck and Company, Inc., Rahway, N.J.).

The pharmaceutical compositions or immuno-therapeutic methods according to the present invention may be used for the treatment of degenerative disorders or cancers. For example the compositions and methods may be employed in the therapy of diagnosed cancers in order to eliminate the tumour cells from the affected organism. As well primary tumours as metastases or disseminated tumour cells within an organism may be targets to the therapeutic compounds and methods disclosed herein.

Furthermore the compositions and methods of the invention may be employed in the treatment of pre-neoplastic conditions. In this case the pre-neoplastic cells or tissues may be directly addressed by the immuno-therapeutic compositions or methods, or may be hindered from evolving into neoplastic or dysplastic conditions. For example in this case the pre-neoplastic condition may be treated preventively. By the vaccination the immune response may be elicited, so that emerging neoplasms may be destroyed.

The methods and compositions according to the present invention may also be used for the prevention and therapy of tumors. For this purpose a vaccination of a population of organisms or of subgroups of said population may be performed. The subgroups may be built by suitable parameters such as hereditary predisposition for the emergence of degenerative disorders, exposure to factors, that increase the risk of being affected by said disorders etc.

The present invention provides compositions and methods for enhanced detection and treatment of tumors. The invention provides peptides derived from proteins, shewing an altered expression in association with tumors. These peptides may thus be employed as a preventive vaccine, immuno-therapeutic agents against said dis orders or may serve as diagnostic tools. Furthermore the invention also provides sets of (poly)peptides useful for the treatment of particular types of disorders. The use of sets of tumor associated polypeptides for the immunotherapy according to the present invention provides the means for a reliable treatment of tumors reducing the risk of escape of several tumour cells or of a population of tumour cells from being addressed by the therapy. Thus the method of the invention reduces the risk of recurrence of tumours after immuno-therapy employing CTLs, T-helper cells and possibly specific antibody producing B-cells raised against tumor associated polypeptides characteristic for tumour cells.

The following examples are given for illustration of the invention only and are not intended to limit the scope of the invention.

### Example 1: In vitro stimulation of cellular immune response by HLA-A1 restricted peptides

The present experiments were performed in order to determine, whether the peptides according to the present invention are suited to stimulate a cellular immune response. The experiments were performed as follows:

Peptides displaying HLA-A2.1-as well as HLA-A1 and HLA-A3-binding motifs were selected by taking advantage of specific computer programs [(Parker, Bednarek, & Coligan 1994); http://bimas.dcrt.nih.gov/molbio/hla_bind/ and (Rammensee et al. 1999); http://134.2.96.221/scripts/MHCServer.dll/home.htm]. Peptides were purchased from the peptide synthesis unit of the DKFZ. Stock solutions (10mg/ml in DMSO) were stored at -70°C and diluted to 1 mg/ml in PBS before use.

In one representative experiment T2 cells were pulsed with 7 p14ARF specific HLA-A2.1 restricted nonamer peptides(Pos. 51: MVRRFLVTL, Pos. 91: AAVALVLML, Pos. 92: AVALVLMLL, Pos. 97: LMLLRSQRL, Pos. 8: HIMGRGRCV, Pos. 85: AAPGAPAAV, Pos. 87: GAPAAVALV; 50µg/ml peptide) and 5µg/ml β2-microglobulin (Sigma; Deisenhofen, Germany) overnight at 37°C. The expression of HLA-A2.1 was then analysed by flow cytometry using mAb BB7.2 followed by incubation with FlTC-conjugated (ab')2 goat anti-mouse Ig (Vonderheide et al. 1999). Peripheral blood was obtained from healthy HLA-A2.1 donors and collected in heparinized tubes. PBMNC were isolated by Ficoll-density gradient centrifugation. Whole CD3+ T cells were isolated from PBMNC by magnetic depletion of non-T cells using the MACS Pan T Cell Isolation Kit (Miltenyi; Bergisch Gladbach, Germany) according to manufacturer's instructions. Preparations contained at least 97% of CD3+ cells as assessed by immunophenotypic analysis.

CD40 Bs of a HLA-A2.1 donors were incubated with the same peptides listed above (10µg/ml) and human β2-microglobulin (3µg/ml; Sigma) in serum-free lscov's DMEM medium for one hour at room temperature, washed twice to remove excess of peptide, were irradiated (30Gy) and added to purified CD3+ autologous T cells (>97% CD3+) at a ratio of 4:1 (T:CD40 Bs) in lscov's MEM containing 10% human AB-serum, supplements (1:100) and hlL-7 (10ng/ml, R&D). Cells were plated at a density of 2 × 10⁶ T cells/well in 1ml of medium. After three days in culture they were fed with 1 ml complete medium. For restimulation of T cells, this was repeated weekly. IL-2 was first given at day 21 (10lU/ml R&D), also at day 24 and from day 28 on only hlL-2 was used instead of hlL7.

ELISpot assays were performed as described elsewhere (Meyer et al. 1998). Briefly, nitrocellulose-96-well plates (Multiscreen; Millipore, Bedford, USA) were covered with mouse anti-human IFN-g monoclonal antibody (Mabtech, Sweden) and blocked with serum containing medium. Varying numbers of effector cells were plated in triplicates with 3,5x10⁴ peptide-loaded T2 cells per well as targets. After incubation for 18h, plates were washed, incubated with biotinylated rabbit anti-human IFN-g second antibody, washed again, incubated with streptavidin coupled alkaline phosphatase, followed by a final wash. Spots were detected by incubation with NBT/BCIP (Sigma) for 45min, reaction was stopped with water, after drying spots were counted using the KS-ELlSpot reader (Zeiss Kontron; Göttingen, Germany).

The analysis shows, that the used peptides are suited to raise an immune response. Immunogenic Peptides deduced from the p14ARF sequence thus may be used to raise immune response for example in the course of vaccinations according to the present invention.

### Example 2: Cytotoxicity assay directed against cells displaying peptides.

CTL bulk cultures and/or CTL clones obtained according to the method described in Example 1 were tested for their cytotoxicity as follows:

To identify the cytolytic potential of CTL clones one HLA matched HLA-A2.1 positive cancer cell line (CASKI) expressing p14ARF as well as p14ARF transfected, p14ARF negative cell line (SW480) of the same HLA haplotype was analysed.

After selection and expansion of the transfected cell lines at least two stably transfected sub-cell lines were available. These cell lines were used in cytotoxicity assays, wherein negative controls were p14ARF negative cell lines (SW480, HCT116), one p14ARF positive cell line of different HLA-haplotype (SIHA) and/or the respective untransfected cell line (SW480). It could be shown, that the transfected cell lines as well as p14ARF positive cell lines of the correct haplotype were lysed by the bulk cultures and/or pooled clones of CTLs. The reactivity was tested at different target cell to effector cell ratios. In average around 20%-30% of the target cells were lysed in the assays. The control cells were always lysed at a significantly lower percentage.

These results shall be representative for the results related to the other peptides (HLA-A1 and HLA-A3 restricted), which rendered similar rates of lysis.

The experiments show, that the peptides disclosed herein may raise immune response. The peptides may furthermore be applied for the detection of the presence of cytotoxic T-cells directed against a particular frameshift peptide.

### Example 3: Screening for Antibodies directed against peptides derived from lNK4a encoded proteins in patient samples

Serum of 25 patients with diagnosed cervical dysplasias was tested for the presence of antibodies to peptides derived from p14ARF as disclosed herein. As a control the serum of 50 normal individuals was tested.

For the test synthetic peptides representing immunogenic regions of the p14ARF protein sequence associated with cervical dysplasias were spotted onto nylon membranes. The nylon membranes were subsequently incubated for one hour in phosphate-buffered saline (PBS) with 5% milk powder for blocking unspecific membrane binding. After washing the membranes 3x with PBS, the membranes were incubated with the test and control sera. The sera were diluted 1:1.000 in PBS/0,5% milk powder and incubated overnight with gentle shaking. Subsequently the sera were removed, and membranes were washed three times in PBS before they were incubated with a polyclonal alkaline phosphatase conjugated goat anit-human lgG antibody for one hour. Thereafter, the membranes were washed repeatedly with PBS/0,05% TWEEN20 before staining reaction was developed using nitroblue tetrazolium chloride and bromochoro-indoyl-phosphate (SigmaAldrich) in Tris-buffered saline (TBS). Binding of human antibodies specific for individual p14ARF specific polypeptides thus was made visible by color-deposit on the respective membrane.

The results show, that in samples of tumour patients antibodies directed against at least one p14ARF specific peptide were present.

This illustrates, that the method according to the present invention may be used for diagnosis of tumors such as cervical dysplasias.

## Claims

1. An immunogenic peptide derived from a cell cycle regulatory protein encoded by an alternative reading frame of the INK4a gene locus, for use in detection and treatment of tumors.

2. The immunogenic peptide according to claim 1, wherein the peptide is selected from a group comprising:
a. a peptide, which may be predicted as being immunogenic, from the amino acid sequence of the cell cycle regulatory protein;
b. an HLA-A3 restricted nonamer peptide;
c. an HLA-A2 restricted nonamer peptide;
d. an HLA-A*0201 restricted nonamer peptide;
e. or a 15-mer peptide

3. An immunogenic peptide according to claim 2, wherein the peptide is selected from a group comprising the sequences given in SEQ IDs No. 1-23.

4. Use of one or more immunogenic peptides according to any one of the preceding claims for treatment of tumors.

5. Use according to claim 4, wherein the treatment is selected from a group comprising curative and preventive immunotherapy.

6. Use according to claim 5, wherein the immunotherapy is vaccination therapy.

7. Use according to any one of the claims 4-6, wherein the tumor is selected from a group comprising benign or malignant tumors, carcinomas, sarcomas, leukemias, lymhomas and dysplasias.

8. Use according to claim 7, wherein the tumor is selected from a group comprising cervical cancer, lung cancer, gastric cancer, and colon cancer.

9. Use according to any one of the claims 4-8, wherein furthermore one or more other peptides derived from tumor associated proteins are used.

10. A binding agent directed against the immunogenic peptide according to any one of the claims 1-3, selected from a group comprising
a. a monoclonal antibody;
b. a mini-antibody;
c. an antigen binding fragment;
d. an antigen binding peptidomimetic molecule;
e. or a polyclonal antibody
for use in detection and treatment of tumors.

11. A pharmaceutical composition comprising one or more peptides according to any one of the claims 1-3 and/or one or more binding agents according to claim 10 for the treatment of tumors, wherein the tumor is selected from a group comprising cervical cancer, lung cancer, gastric cancer, and colon cancer and the treatment is selected from a group selected from a group comprising curative and preventive immunotherapy and vaccination therapy.

12. The pharmaceutical composition according to claim 11 comprising furthermore one or more additional peptides derived from proteins, which show non wild-type expression in tumors.

13. The pharmaceutical composition according to claim 12, wherein the additional peptides are derived from proteins selected from a group comprising p16^{lNK4a}, HPV E6, HPV E7, HPV E2 HPV E4, HPV L1, HPV L2, p27, p21, p15, p19, p53, pRb, MDM2 and peptides encoded by the genes disclosed in the documents WO9904265A2, WO014971 6A2, WO0055633A2 and/or WO01 42792A2.

14. A method for detection of immunological entities specifically recognizing the peptides according to any one of the claims 1-3 in individuals comprising the steps of
a. obtaining a sample from the individual;
b. contacting the sample with a binding agent binding to said immunological entities selected from a group comprising
i. a binding agent directed against said immunological entities
ii. a binding agent directed against complexes of the immunological entities together with the respective peptides
iii. at least one peptide according to any one of the claims 1-3,
wherein said contacting is performed in a way, that binding of the immunological entities to said binding agents gives rise to a detectable signal;
c. and assessing the presence or absence and/or the level of immunological entities in said sample from the presence or absence and/or the level of detectable signal.

15. The method according to claim 14, which is used for purposes selected from a group comprising
a. monitoring in the course of a therapy using peptides according to claim 1-3;
b. monitoring in the course of the application of a pharmaceutical composition according to claim 11-13; and
c. monitoring in the course of a use according to any one of the claims 4-9.

16. The method according to claim 14, which is used for the diagnosis and monitoring of the disease course of tumors.

17. The method according to any one of the claims 14 - 16, wherein the sample is selected from a group comprising secretions, smears, body fluids, serum, blood, plasma, urine, semen, stool, bile, sputum, biopsies, cell- and tissue-samples, resection samples of tumors, tissue samples prepared by endoscopic means and needle biopsies of organs.

18. A kit for performing the method according to claims 14 to 17 in the course of research studies or in the course of diagnostic procedures.

19. The kit according to claim 18 comprising at least one item selected from a group comprising one or more binding agents according to claim 10, one or more peptides according to claims 1-3 and one or more peptides derived from proteins associated with tumors.
